# EUROPEAN PATENT APPLICATION

(11) **EP 3 868 886 A1**
(43) Date of publication of application: **25.08.2021**
(21) Application number: 20164752.6
(22) Date of filing: 22.03.2020
(51) Int. Cl.: C12N 15/86, C12N 7/02, B01D 15/38, A61K 39/23

(54) **A METHOD FOR SEPARATION OR DEPLETION OF EMPTY AAV CAPSIDS FROM FULL AAV CAPSIDS**

(30) Priority: 21.02.2020 EP 20158833
(71) Applicant: BIA SEPARATIONS d.o.o., 5270 Ajdovscina (SI)
(72) Inventor: CERNIGOJ, Urh, 5270 Ajdovscina (SI); GORICAR, Blaz, 3314 Braslovce (SI); DOLENC, Darko, 1360 Vrhnika (SI); GAGNON, Peter S., 5270 Ajdovscina (SI)
(74) Representative: dompatent von Kreisler Selting Werner - Partnerschaft von Patent- und Rechtsanwälten mbB

(57) **Abstract**

A method for the separation or depletion of empty AAV capsids from full AAV capsids in an aqueous mixture comprising empty and full AAV capsids, wherein the mixture is contacted with a primary amino groups bearing solid phase surface in a first alkaline milieu whereby
(i) full AAV capsids bind to the solid phase surface whereas empty AAV capsids at least partially do not bind to the solid phase surface,
or
(ii) both full and empty AAV capsids bind to the solid phase surface, and subsequently the empty AAV capsids are at least partially eluted by means of a second alkaline milieu of a pH value higher than the pH value of the first alkaline milieu, with the proviso that the second alkaline milieu does not elute full AAV capsids from the solid phase surface.

## Description

The invention pertains to a method for separation or depletion of empty AAV capsids from full AAV capsids employing solid phase extraction.

Preparations of recombinant adeno-associated virus (AAV) are commonly populated by two subpopulations of capsids. The desired capsids are properly filled with their intended therapeutic DNA payload. These are referred to as full capsids. Impure preparations also contain capsids that are not filled with their intended DNA load. These are referred to as empty capsids. Some preparations may contain 90% or more empty capsids, others less than 50%. Current clinical guidelines recommend that the content of empty AAV capsids be reduced to less than 10%. This can be achieved by ultracentrifugation methods, but the equipment and process are difficult to scale up and prone to failure due to trivial operator errors. Chromatography methods are regarded by many as advantageous over centrifugation.

A chromatography method for separating empty AAV capsids from full AAV capsids is known [1-3]. Employing strong anion exchangers and eluting them with a salt gradient at alkaline pH has proven to support adequate reduction of empty AAV capsids for many AAV serotypes. However, results are sometimes inadequate in one or more respects. First, the degree of separation tends to be limited. Cases are known where baseline resolution is achieved, meaning the peaks corresponding to the empty and full AAV capsids are completely separated, but these are the exception. The respective elution peaks typically overlap to a degree, sometimes substantially, sometimes almost entirely. This makes it necessary to sacrifice the full AAV capsids that overlap with empty AAV capsids in order to eliminate enough of the empty capsids. Full capsid recovery is thereby compromised.

A related compromise also pertains to the limited separation achieved by strong anion exchangers: it imposes a burden on industrial scale use of the technique. Many users prefer to employ step gradient elution with large scale chromatography techniques since they are simpler and they save money on equipment, buffer preparation, fraction collection and analysis. Step gradients are unfortunately burdened by reproducibility problems since every single process variable must be invariant from processing lot to processing lot. Among other variables, buffers must be of precisely identical composition with respect to both pH and conductivity, chromatography media must be of precisely identical composition, processing temperature must be identical, etc. None of these parameters are invariant in actual practice and results inevitably vary with respect to recovery of full AAV capsids and/or the extent to which empty AAV capsids are eliminated.

Linear gradient elution supports better reproducibility. An elution profile may shift slightly due to variation in materials or conditions but the relative relationship between the eluting full and empty capsid peaks is insulated from these variations and the separation is maintained. However, manufacturing scale equipment to support linear gradient elution is more expensive, requires more training, more buffers, analysis of more fractions, and more time than step gradients.

A third limitation pertains to the extreme chemical conditions under which empty-full separation is conducted on strong anion exchangers. Required pH is often pH 9.0 or higher and sometimes up to pH 10.0 or higher [1-3]. Often, the higher the pH, the better the separation. Chemical stress created by pH values above pH 9.0 is unfortunately known to decrease stability of many biologics and the higher the pH above 9.0, the higher the level of chemical stress. Chromatograms of highly purified AAV capsids often show peaks in addition to empty and full AAV capsids that are interpreted to represent partially dissociated capsids and free capsid proteins. DNA released by damage to previously intact full AAV capsids may also be evident.

The term strong anion exchanger is understood to pertain to chromatography media bearing quaternary amine ligands. The adjective "strong" particularly refers to their ability to maintain their full electrostatic charge over a wide range of pH values, such as from about pH 2.0 to pH 12.0. Commercial product names commonly include Q, QA, QAE, referring to quaternary, quaternary amine, quaternary aminoethyl, respectively. Other names for strong anion exchangers include TEAE (triethylaminoethyl) or TMAE (trimethylaminoethyl), which correspond to the same degree of amine derivatization but a different naming convention. TEAE and TMAE still describe quaternary amine ligands.

Examples of less derivatized amino chromatography ligands are widely known under names such as DEAE (diethylaminoethyl). DEAE represents a tertiary amino ligand. Tertiary amino ligands are examples of so-called weak anion exchangers because they maintain their full charge over a relatively restricted range of pH values. DEAE ligands begin to lose charge at pH values as low as 7.5. They have lost most their charge at pH 9.0, and essentially all their charge at pH 12. Low charge at pH 9.0 translates to low capacity for AAV which compromises productivity. It also suggests that weaker anion exchange ligands such as secondary amino or primary amino ligands are likely to give even less favorable results compared to quaternary amino ligands. It is notable in this regard that weak exchangers like DEAE are seldom discussed for separation of empty and full AAV capsids.

Anion exchangers are most commonly eluted at a fixed pH using salt gradients, such as created by increasing the concentration of sodium chloride. They are sometime eluted with decreasing pH gradients. The charge on a strong exchanger remains constant within the range of about 2 to 12 but the charge on proteins changes. Protein electronegativity becomes weaker with descending pH while protein electropositivity becomes stronger. Decreasing electronegativity means decreasing attraction to the positively charged anion exchange surface. Increasing electropositivity translates to increasing repulsion from the positively charged anion exchange surface. The two phenomena work cooperatively to achieve elution with descending pH. Exposing a strong anion exchanger to an ascending pH gradient causes bound components to bind more strongly.

Cation exchangers are used extensively for AAV purification but most often using salt gradient elution that causes the empty and full AAV capsids to elute together in a single peak. They can also be eluted with pH gradients but since cation exchangers are electronegative where anion exchangers are electropositive, gradient elution of cation exchangers requires ascending pH. Elution of cation exchangers with ascending pH gradients enables them to achieve a degree of separation between empty and full AAV capsids but the degree of separation is inferior to the separation provided by salt elution of strong anion exchangers.

### Summary

The present invention is based on the surprising finding that a separation of full AAV capsids from empty AAV capsids can be achieved by means of a solid phase modified with primary amino groups. The method of the invention is able to overcome several practical limitations associated with the known method of removing empty AAV capsids using strong (quaternary amine) anion exchangers eluted with ascending salt gradients at fixed alkaline pH [1-3]. The primary enabling feature of the method of the invention is the surprising finding that the majority of empty AAV capsids could be removed selectively by increasing pH in the alkaline range despite the absence of excess salt. This is the opposite of how empty AAV capsids behave with known anion exchange methods where capsid binding is strong at alkaline pH in the absence of excess salt [1-3] and becomes even stronger at higher pH values. The term "excess salt" in this context refers to salts beyond the buffering components used to control the pH of the operating solutions, such as the sodium chloride (NaCI) or others which may be added to a Tris buffer, or bis-tris-propane buffer, or other buffer to achieve elution of a bound component.

In a general aspect the invention is related with a method for separation or depletion of full AAV from empty AAV capsids from an aqueous mixture containing full and empty AAV capsids employing solid phase extraction wherein the aqueous mixture containing full and empty AAV capsids is contacted with a primary amino group bearing solid phase.

In another aspect the invention is related with a method for the separation or depletion of empty AAV capsids from full AAV capsids in an aqueous mixture comprising empty and full AAV capsids, wherein the mixture is contacted with a primary amino groups bearing solid phase surface in a first alkaline milieu whereby
(i) full AAV capsids bind to the solid phase surface whereas empty AAV capsids at least partially do not bind to the solid phase surface, or
(ii) both full and empty AAV capsids bind to the solid phase surface, and subsequently the empty AAV capsids are at least partially eluted by means of a second alkaline milieu of a pH value higher than the pH value of the first alkaline milieu, with the proviso that the second alkaline milieu does not elute full AAV capsids from the solid phase surface.

In one embodiment of the invention, the pH value of the first alkaline milieu can be pH > 7, in particular pH > 7 to pH 8.

In another embodiment of the invention, the pH value of the second alkaline milieu can be greater than the pH value of the first milieu.

In yet another embodiment of the invention, the solid phase surface may be contacted with a third alkaline milieu having a pH value greater than the pH value of the second milieu after an at least partial elution of the empty AAV capsids.

In still another embodiment of the invention, the solid phase surface can be contacted with a forth alkaline milieu having a pH value less than the pH value of the third or second alkaline milieu and a higher salt concentration compared to the salt concentration of the first, second or third alkaline milieu, after at the least partial elution of the empty AAV capsids.

According to the invention the pH value of the second alkaline milieu particularly may be in the range of pH 8.0 to pH 9.0, pH 8.1 to pH 8.9, pH 8.2 to pH 8.8, pH 8.3 to 8.7, or pH 8.4-8.6.

According to the invention the pH value of the third alkaline milieu particularly may be in the range of pH 8.5 to pH 10.5, or pH 8.5 to 10.0, or pH 8.5 to 9.5.

The overlap in the pH range values of the first, second or third pH value is virtual. The skilled person readily knows that if the pH value of the first alkaline milieu has been selected to be pH 8 (the range spans of pH > 7 to pH 8) and the respective pH value of the second alkaline milieu shall be chosen from the range of pH 8.0 to pH 9.0, this means that the pH value of the second alkaline milieu must not be the lowest value of the pH range, i.e pH 8, but a higher one e.g. 8,1 or 8.2 and so on. The same considerations are true with respect to the virtual overlap of the pH ranges of the second and third alkaline milieus.

In another embodiment of the method of the invention comprise a salt concentration up to 1 M corresponding to the concentration of an alkali metal salt, such as NaCl, in particular 1 mM to 1,000 mM, or 2.5 mM to 250 mM, such as 2.5 mM, or 5 mM, or 12.5 mM, 25 mM, or 50 mM, or 100 mM, or 150 mM, or 200 mM, or 250 mM. If a different alkali metal salt than sodium salt or a mixture of different alkali metal salts shall be used the person skilled in the art is able to determine the respective relation with regard to the ionic strength, conductivity and other effects of the respective "pure" sodium metal salt concentration.

In a particular embodiment of the invention the alkaline milieus may comprise magnesium salts at a concentration of the magnesium ions in the range of 1.0 mM to 5.0 mM, in particular 1.5 mM to 3.0 mM, or 2.0 mM to 2.5 mM.

An advantageous aspect of the present invention is the fact, that the full and empty AAV capsids may be of any serotype, in particular selected from the group consisting of natural and recombinant serotypes, chimeric (mixed), and combinations thereof.

The method of the invention may be applied in any device which is customary in the art, e. g. the primary amino groups bearing solid phase surface can be arranged in a chromatography device. Typically, the primary amino groups bearing solid phase surface can be a monolith, a column of packed particles, a column of packed nanofibers, a membrane adsorber, or a hydrogel.

Subject matter of the present invention is also the use of a solid phase extraction material having a solid phase surface bearing primary amino groups for the separation or depletion of empty AAV capsids in an aqueous mixture comprising empty and full AAV capsids.

The method of the invention may be preceded, or followed, or both by additional processing steps to reduce empty capsid content to a greater degree than can be achieved by any one of the processing steps by itself. Additional processing steps presently known to offer utility for reducing the amount of empty capsids include ion exchange chromatography with a quaternary amine ion exchanger and density gradient ultracentrifugation. In one embodiment, the combination of the invention with ion exchange chromatography on a quaternary anion exchanger might produce a full capsid fraction with a sufficiently low concentration of empty capsids that no additional processing would be required to remove empty capsid. The two steps might be performed in any sequence. In another embodiment, the method of the invention can be followed by density gradient ultracentrifugation. In another embodiment, the combination of the invention with ion exchange chromatography on a quaternary amine anion exchanger can be followed by density gradient ultracentrifugation.

No theory has yet been developed concerning the specific mechanism or mechanisms by which the invention works; only that it is fundamentally distinct from known cation exchange or anion exchange chromatography, and that it achieves superior separation of empty AAV capsids from full AAV capsids compared to the standard of salt elution on strong anion exchangers. Superiority of separation refers to any combination of three following attributes: The separation can be conducted under milder chemical conditions that favor conservation of full capsid stability, and/or the degree of separation between empty and full AAV capsids is increased, and/or the degree of separation is sufficient to make step gradient elution a viable approach.

The method may be employed for purpose of performing analytical or preparative applications. The method is applicable to all AAV serotypes. The specific chromatography conditions and degree of separation between empty and full AAV capsids may differ among serotypes, and among different recombinant constructs within a serotype. This is a well-known feature of empty-full separation conducted with the known method of ion exchange chromatography and simply reflects natural variation of capsid surface chemistries among different AAV serotypes.

### Brief Description of The Figures

Fig. 1 depicts results of a comparative separation of empty AAV capsids from full AAV capsids by salt elution of a quaternary amino solid phase versus a primary amino solid phase.
Fig. 2 depicts results of a comparative separation of empty AAV capsids from full AAV capsids by salt elution of a quaternary amino solid phase versus elution by ascending pH gradient primary amino solid phase.
Fig. 3 depicts results of an equilibration and loading of a primary amine column under conditions where empty and full AAV capsids both bind, empty AAV capsids are removed with a second wash step, and full AAV capsids are eluted with an ascending pH gradient.
Fig. 4 depicts results of an equilibration and sample loading of a primary amine column under conditions where empty AAV capsids do not bind followed by step gradient elution of full AAV capsids with increasing salt while reducing pH.
Fig. 5 to Fig. 10 depict results of a series of experiments. A sample containing empty and full AAV8 capsids was applied to a primary amino groups bearing solid phase at pH 8.0. In each experiment, the loaded column was washed with buffer at a higher pH and then eluted with an increasing pH gradient, ending at pH 9.5.
Fig. 11: Relative recovery of empty AAV capsids and full AAV capsids from the gradient is presented graphically.
Fig. 12: The influence of magnesium ions or their absence on the elution of empty and full AAV capsids is illustrated by comparative chromatograms.

### Detailed Description of The Invention

The term "primary amine-bearing solid phase" refers to a solid phase dominantly or exclusively bearing primary amine ligands on its surfaces, where the term primary amino ligand describes a nitrogen atom linked by single covalent bonds to each of two hydrogen atoms, and also linked by a single covalent bond to a carbon atom. Secondary amines should be absent from the surface of the solid phase or in a minority. Tertiary and quaternary amines should be absent or represent a small minority. Negatively charged residues should be absent. Uncharged hydrophobic or hydrogen bonding residues may be present. The primary amino ligand may be covalently linked directly to a solid phase through the ligand carbon atom. Alternatively, the primary amino ligand may be linked indirectly to the solid phase by covalent attachment of the ligand's carbon atom to a so-called spacer arm that is covalently linked to the solid phase. The primary amino ligand may also be part of a polymeric structure covalently linked to the solid phase. The solid phase may be a chromatographic solid phase in the form of one or more porous membranes, one or more fibers, one or more porous or non-porous particles, or a monolithic solid phase, including monoliths synthesized from a single polymer mixture, or monoliths synthesized first as a macro-skeleton with a secondary ligand-bearing polymer phase synthesized on top of it. Any of the above solid phase materials may be provided in a housing to facilitate performance of chromatography. Chromatographic solids phases in housings are commonly referred to as chromatographic devices and often as columns. Chromatographic solid phases bearing primary amines are known and available commercially. One example is marketed under the name Toyopearl NH2-750F (Tosoh Biosciences), where "NH2" refers to the primary amine [https://www.separations.eu.tosohbioscience.com/solutions/process-media-products/by-mode/ion-exchange/anion-exchange/toyopearl-nh2-750f]. Marketing materials indicate that the primary amine ligand is in the form of a polyamine, meaning that it is a polymer with repeating primary amine subunits affixed covalently to the solid phase. Another example is marketed under the name Sartobind STIC PA (Sartorius), where "PA" refers to the primary amine [https://www.sartorius.com/shop/ww/en/usd/sartobind-stic®-pa/c/M_Sartobind_STIC_PA]. Marketing materials indicate that the primary amine ligand is in the form of a polyamine, specifically polyallylamine, with repeating primary amine subunits affixed covalently to the solid phase. All major producers of chromatographic solid phases manufacture products with amine derivatives on their surfaces, indicating the requisite knowledge and resources in the field to produce primary amine-bearing solid phases on a regular or experimental basis.

Given that chromatographic solid phases bearing primary amines may not be named in a manner that clearly reveals their composition, it will be useful to have an analytical method to determine if a given product has appropriate characteristics to practice the invention. One simple method for making this determination is to equilibrate the chromatographic solid phase in question with a buffer such as 10 mM Tris, 10 mM bis-tris-propane, 15 mM NaCl, pH 7.2, apply a sample consisting of protein standards for isoelectric focusing, wash the solid phase with equilibration buffer, then apply a 50 column volume (CV) linear gradient beginning with equilibration buffer and ending at 10 mM Tris, 10 mM bis-tris-propane, pH 9.5, with a 10 CV hold at the endpoint buffer. Elution of proteins across the linear gradient will be an indication that the solid phase in question is a primary amine-bearing solid phase. An experimental control consisting of a quaternary amine-bearing solid phase may be run in parallel. The proteins will not elute. Protein standards for isoelectric focusing are commercially available from various suppliers including ThermoFisher, Bio-Rad, and Serva, among others.

In some cases, solid phases bearing polyethyleneimine (PEI) may permit elution of proteins with alkaline isoelectric points according to the above qualification test and they may permit a degree of separation between empty and full capsids. Linear PEI is an amine-bearing polymer with the termini occupied by primary amines and with secondary amines at each repeating subunit of the polymer. Branched PEI polymers also bear tertiary amines at the branch points. PEI solid phases are known to enable descending pH gradient separation of some proteins but, as with quaternary amine solid phases, only ascending pH gradient elution is known.

The term "equilibrated" or "equilibration" refers to a chemical conditioning step having been performed on the solid phase and/or on the sample to create a specific chemical environment. Solid phases are customarily conditioned by exposing them to a buffer that embodies the desired pH, salt composition, and salt concentration. Samples are customarily conditioned by titration of pH, sometimes by dilution to reduce the salt concentration, and sometimes by buffer exchange techniques including by chromatography, or by dialysis, or by diafiltration with tangential flow filtration membranes. In the present case, equilibration conditions can be set so the solid phase binds both empty AAV capsids and full capsids, or they can be set so that a subset of empty capsids, potentially including the majority or all of the empty capsids, fail to bind to the solid phase during sample loading. All of these approaches and the criterial for choosing one or another have been well known in the art for decades.

The full and empty AAV capsids in any given sample may belong to any serotype, including natural and recombinant serotypes, including chimeric (mixed) and novel serotypes. It will be understood that capsids of different serotypes have different charge properties, including different levels of charge distinction between empty AAV capsids and full capsids, and that such differences may also be apparent among different recombinant constructs within a serotype. This is important because it means the specific behavior of any given preparation may vary on the primary amino solid phase, as they also vary on strong anion exchangers. In turn, that means that some degree of optimization is likely to be required when applying the method to any previously untried AAV sample, as is also the case with the known method of separating empty and full AAV capsids with salt gradients on strong anion exchangers. The sample may be in the form of a cell culture harvest, a cell lysate, a partially purified preparation such an elution fraction from an affinity chromatography column, a partially purified fraction eluted from an ion exchange chromatography column, a partially purified fraction eluted from a hydrophobic interaction chromatography column, or capsid mixtures from any other purification method or combination of methods employed to process AAV.

The term "loaded" refers to the process of bringing the equilibrated sample into contact with the equilibrated primary amino solid phase. This is usually done with chromatography devices by causing the sample to pass through the device by means of an external force, such as by gravity or by pumping.

The term "adsorption" refers to the process of binding a biological product to a chemically complementary surface. Complementarity in the present case is understood to involve electrostatic charge. The negative electrostatic charge on the surface of AAV capsids mediates their adsorption to the surface of a solid phase surface that has been rendered electropositive by means of covalent immobilization of primary amine resides. Adsorption of biological products to solid phases used for chromatography is most often referred to as "binding".

The term "selective adsorption" refers to the application of conditions that permit adsorption of at least one species while preventing adsorption of one or more other species. In the present case, operating pH can be adjusted into the alkaline range to prevent adsorption of undesired empty AAV capsids while permitting the desired full AAV capsids to be adsorbed.

The term "desorption" refers to the process of releasing a biological product from a chemically complementary surface to which it has been previously adsorbed. Such desorption may be achieved in the present case by a variety of means, including but not limited to reducing the electronegativity of the biological product (AAV), for example by reducing pH; or by reducing the electropositivity of the solid phase, for example by increasing pH; or by interfering with electrostatic interactions by introduction of a competing substance, such as a salt; or any combination of these approaches. Desorption of biological products from chromatography media is most often referred to as elution.

The term "selective desorption" refers to situations in which one adsorbed species is released from the solid phase surface by a change in the conditions that leaves one or more additional species still adsorbed, and then a yet different set of conditions causes release of a different species from the solid phase surface. The changes in conditions may be conducted in steps. Changes in conditions may also be made in a continuous manner, causing weakly bound species to be desorbed early in the continuum while strongly bound species elute later in the continuum, ideally well separated from each other.

The term "washed" refers to a process of exposing the loaded column to clean buffer for the purpose of displacing unbound species from the solid phase. The term "rinsed," in the present context, has the same meaning. In the most basic case, the wash buffer has the same formulation as the equilibration buffer. In more complex configurations, a wash buffer may have the additional role of displacing a subset of weakly bound contaminants from the solid phase so that they can be removed before the desired product is eluted. Or there may be more than one washing step where the first employs conditions identical to the equilibration buffer but the second employs conditions that displace a subset of weakly bound contaminants from the solid phase so that they can be removed before elution.

The term "elution" or "eluted" refers to a process of changing the chemical environment in which the solid resides to cause dissociation of the interaction between the primary amino solid phase and the species that remained bound after the load and wash steps.

With empty AAV capsids removed from the solid phase, full AAV capsids may be eluted by any of a variety of approaches. As noted above, full AAV capsids may be eluted by further increasing pH in the absence or excess salts. With empty AAV capsids removed, full AAV capsids may be eluted alternatively by an increase in pH in the presence of salts. With empty AAV capsids removed, full AAV capsids may be eluted alternatively by introducing excess salts while pH is held constant. With empty AAV capsids removed, full AAV capsids may be eluted alternatively by introducing excess salts while pH is reduced.

The following series of general non-limiting descriptions of basic method options illustrates variations in how the method may be performed and provides a platform for more detailed discussion of operational variables. It is understood that the buffer conditions mentioned in each of these scenarios are intended to provide a general idea of how the method may be practiced and that optimization of buffer formulations will be required to accommodate capsid mixtures from different serotypes, or even different recombinant constructs within a serotype.

In one embodiment exploiting only elution with an ascending pH gradient, a primary amino solid phase in the form of a chromatography device such as a monolith, is equilibrated to a pH value close to neutrality, such as 20 mM Tris, 20 mM bis-tris-propane, pH 7.5 ± 0.2. A sample containing a mixture of empty and full AAV capsids is equilibrated by buffer exchange to 20 mM Tris, 20 mM bis-tris-propane, pH 7.5 ± 0.2. The chromatography device is then eluted with a linear pH gradient from the equilibration buffer to an endpoint buffer of 20 mM Tris, 20 mM bis-tris-propane, pH 9.5 ± 0.2 over 20 device-volumes, or over 50 device-volumes, or over 100 device volumes, where the number of device volumes is used as a means of adjusting the rate at which pH changes during the gradient, in other words the gradient slope. This approach may particularly be used for analytical purposes because the entire empty capsid and full capsid populations are eluted within the gradient. It is also likely to be valuable as starting point for developing preparative use conditions. It may also be used for preparative applications but with awareness that this approach potentially exposes the full AAV capsids to a range of pH values that may have adverse effects on capsid stability.

In an embodiment exploiting only elution with an ascending salt gradient, a primary amino solid phase in the form of a chromatography device such as a monolith, is equilibrated to a slightly alkaline value, such as 20 mM Hepes pH 7.5 ± 0.2. A sample containing a mixture of empty and full AAV capsids is equilibrated by buffer exchange to the same conditions. The chromatography device is then eluted with a linear sodium chloride gradient from the equilibration buffer of mM Hepes pH 7.5 ± 0.2 to an endpoint buffer of mM Hepes, 200 mM NaCl, pH 7.5 ± 0.2 over 20 device-volumes, or over 50 device-volumes, or over 100 device volumes, where the number of device volumes is used as a means of adjusting the rate at which pH changes during the gradient, in other words the gradient slope. This approach may also be suited to analytical use since, as above, all of the empty and full capsid bind to the primary amino solid phase during sample loading. The degree of separation between empty and full AAV capsids will generally be inferior to elution with an ascending pH gradient but still superior to fractionation by a strong anion exchanger eluted with a salt gradient at fixed pH.

In a closely related embodiment exploiting only elution with an ascending salt gradient, a primary amino solid phase in the form of a chromatography device such as a monolith, is equilibrated to a mildly alkaline pH value, such as 10 mM Tris, 10 mM bis-tris-propane, pH 8.7 ± 0.2. A sample containing a mixture of empty and full AAV capsids is equilibrated by buffer exchange to the same conditions. The chromatography device is then eluted with a linear sodium chloride gradient from the equilibration buffer of 10 mM Tris, 10 mM bis-tris-propane, 100 mM NaCl, pH 8.7 ± 0.2. over 20 device-volumes, or over 50 device-volumes, or over 100 device volumes, where the number of device volumes is used as a means of adjusting the rate at which pH changes during the gradient, in other words the gradient slope. This approach may also be suited to analytical use since, as above, all of the empty and full capsid bind to the primary amino solid phase during sample loading. The degree of separation between empty and full AAV capsids will be better than salt gradients at lower fixed pH values and generally superior to fractionation by a strong anion exchanger eluted with a salt gradient at fixed pH, but still generally inferior to elution with an ascending pH gradient on a primary amino solid phase.

In an embodiment exploiting mixed pH-salt elution, a primary amino solid phase in the form of a chromatography device such as a monolith, is equilibrated to a pH value close to neutrality, such as 20 mM Hepes, pH 7.0 ± 0.2. A sample containing a mixture of empty and full AAV capsids is equilibrated by buffer exchange to 20 mM Hepes, pH 7.0 ± 0.2. The sample is loaded onto the chromatography device. Under these conditions, both empty particles and full particles bind to the solid phase. The device is washed with equilibration buffer to remove unbound species. Empty AAV capsids are washed/eluted from the device by changing the chemical environment by exposure to a buffer with a higher pH, such as 20 mM Tris, pH 8.5 ± 0.2. This buffer is flowed through the chromatography device until the UV absorbance of the column effluent reaches baseline. With the empty AAV capsids removed from the device, the chemical environment is changed again by exposure to a buffer with lower pH but containing excess salt, such as 20 mM Hepes, 50 mM NaCl, pH 7.0. This approach may be advantageous for preparative use because of the gentle full-capsid elution conditions. It is also an example of a step gradient elution format that will be considered advantageous by some users.

In another embodiment exploiting mixed pH-salt elution, a primary amino solid phase in the form of a chromatography device such as a monolith, is equilibrated to an alkaline pH, such as 20 mM Tris, pH 8.5 ± 0.2. A sample containing a mixture of empty and full AAV capsids is equilibrated by buffer exchange to 20 mM Tris, pH 8.5 ± 0.2. The sample is loaded onto the chromatography device. Under these conditions, full particles bind to the solid phase, but empty particles fail to do so. With the empty AAV capsids removed from the device, the chemical environment is changed again by exposure to a buffer with lower pH but containing a small amount of salt such as 20 mM Hepes, 50 mM NaCl, pH 7.0. This approach is procedurally simpler than the previous and therefore attractive for preparative use. However, it may diminish binding capacity for full AAV capsids because the equilibration conditions are close to the threshold that will interfere with full capsid binding.

In an embodiment exploiting pH and salt in an elution of full AAV capsids in another way, a primary amino solid phase in the form of a chromatography device such as a monolith, is equilibrated to a pH value close to neutrality, such as 20 mM bis-tris-propane, pH 8.5 ± 0.2. A sample containing a mixture of empty and full AAV capsids is equilibrated by buffer exchange to 20 mM bis-tris-propane, pH 8.5. The chromatography device is then eluted with a linear salt gradient from the equilibration buffer (pH 7.5) to an endpoint buffer of 20 mM bis-tris-propane, 100 mM NaCl, pH 8.5, over 20 device-volumes, or over 50 device-volumes, or over 100 device volumes, where the number of device volumes is used as a means of adjusting the rate at which pH changes during the gradient, in other words the gradient slope. Preliminary data indicate that salt gradients support separation of empty and full AAV capsids similar to the separation achieved by strong anion exchangers, and under milder conditions compared that strong anion exchangers, but still inferior compared to pH gradients. In most cases, the higher the pH of the salt gradient, the better the resolution between the empty and full capsids.

It will be evident to experienced practitioners of the art that one or more respective elements from any one of the above basic formats may be applied in other formats. For example, elution gradients can be applied that change both pH and salt concentration simultaneously, with both parameters ascending, or one or the other parameter ascending while the other descends; single step elution can be converted to multiple step elution; linear gradient elution can be converted to step elution; linear gradient elution can be applied after a step that substantially reduces the amount of still-bound but does not entirely eliminate them; and linear gradient elution can be applied after loading under equilibration conditions that cause most of the empty AAV capsids not to bind. These and many other variations can be made without departing from the defining elements and true nature of the method.

In one embodiment the primary amino solid phase and sample are equilibrated to an operating pH in the range of pH 5.5 ± 0.2 to pH 8.5 ± 0.2, or pH 6.0 ± 0.2 to pH 8.5±0.2, or pH 6.5 ± 0.2 to pH 8.5±0.2, or pH 7.0 ± 0.2 to pH 8.5±0.2, or pH 7.5 ± 0.2 to pH 8.5±0.2. It is understood that the exact conditions may vary among AAV serotypes and potentially among different recombinant constructs within a serotype. For samples known to tolerate high pH ranges, the pH range may be extended higher. As a general matter, the higher the pH within this range, the fewer empty AAV capsids will bind. Simple experimentation will be required for any previously untried AAV to determine the pH value where empty AAV capsids fail to bind while essentially all the full AAV capsids do bind. The lower the pH within this range, the stronger the full AAV capsids will bind and the higher the binding capacity for full capsids. It will be apparent to persons of experience however that empty AAV capsids may compete with full AAV capsids for binding space and that such competition may limit binding capacity for full capsids. These are routine process optimization issues that are taken into consideration during development of all industrial purification methods.

In some embodiments where empty capsid binding is not suspended at pH 8.5 ± 0.2 and there is a particular desire to avoid higher pH values that may risk capsid stability, salt may be added to the equilibration buffer, such as 2 mM NaCl, or 5 mM NaCl, or 10 mM NaCl, or 20 mM NaCl, or an intermediate or higher concentration. The lowest concentration of salt that achieves suspension of empty capsid binding at a moderate pH will generally be most advantageous. If there is a desire to evaluate the use of higher concentrations to displace empty AAV capsids such as 30 mM, or 40 mM, or 50 mM, or 60 mM, or 70 mM, or an intermediate or higher concentration, they can be evaluated with care to avoid inadvertent displacement and loss of full capsids. This approach can be considered to reduce empty capsid binding particular at acidic, neutral, or very mildly alkaline pH values, if desired.

Many methods of equilibrating samples to the conditions for loading them onto chromatography columns are known to persons of skill in the art. Any of these methods may be employed without altering the true nature of the method. Among these methods are the laboratory-scale method of dialysis, the method of diafiltration with tangential flow filtration membranes, and the method of buffer exchange chromatography. In some cases, adequate sample equilibration may be achieved by titrating the sample to the target pH and, if necessary, diluting the sample with water or a low-salt or non-salt containing buffer.

Equilibration provides an opportunity to prevent binding by empty AAV capsids but the conditions for doing so may weaken the binding of full AAV capsids with the result of reducing the capacity of the solid phase for full capsids. Since a particular goal of preparative separations is to accommodate the largest practical load of the desired full capsids, washing provides a potentially less compromised approach to minimization of empty capsid content before elution. Given the most effective washing conditions, equilibration conditions can be selected that favor high capacity binding of the desired full capsids, which will generally correspond to neutral or lower pH and minimal or no additional salt.

In one embodiment, a primary amine solid phase loaded with a mixture of empty AAV capsids and full AAV capsids is washed with buffer of the same composition as the buffer used to equilibrate the solid phase.

In another embodiment, the primary amine solid phase loaded with a mixture of empty AAV capsids and full AAV capsids is washed with a buffer that has a higher pH than the equilibration buffer. For example, a primary amine solid phase equilibrated and loaded at pH 7.5 might be washed first with buffer of the same composition as the buffer used to equilibrate the solid phase, and then washed again with a buffer of higher pH, such as pH 8.0 ± 0.2, or pH 8.1 ± 0.2, or pH 8.2 ± 0.2, or pH 8.3 ± 0.2, or pH 8.4 ± 0.2, or pH 8.5 ± 0.2, or pH 8.6 ± 0.2, or pH 8.7 ± 0.2. It is understood that the exact conditions may vary among AAV serotypes and potentially among different recombinant constructs within a serotype. In the event that empty AAV capsids are not removed fully by increasing the pH, then salt may be added. For example, to a buffer with a pH of 8.5 ± 0.2, NaCl might be added in an amount corresponding to 2 mM, or 5 mM, or 10 mM, or 20 mM or an intermediate or higher concentration.

In a related embodiment, the approach of washing with different conditions than the sample equilibration pH conditions may be approached in an alternative manner that simplifies the overall method. The sample and the column can be equilibrated to different conditions. Specifically, the sample can be equilibrated to a pH and/or salt concentration that supports the highest capacity binding of full capsids, while the column can be equilibrated to conditions that largely remove empty AAV capsids in advance of the full capsid elution step. For example, the sample may be equilibrated to 50 mM Hepes, pH 7.5 ± 0.2, while the column is equilibrated with 50 mM Tris, pH 8.5 ± 0.2. When the sample is loaded on the column, it will have the effect of re-equilibrating the column, at least partially, to the conditions of the sample. At the end of the sample load, the higher pH conditions of the equilibration/wash buffer will immediately begin to displace empty AAV capsids that became bound during sample application and there will be no need to include a second wash step. Buffer preparation and the overall method will be simplified.

In one embodiment, the full AAV capsids are eluted exclusively by increasing the pH. Depending on the serotype of the AAV capsids, this may consist of a single elution step, or a multiple step gradient elution, or a linear gradient elution to pH 8.6 ± 0.2, or 8.7 ± 0.2, or 8.8 ± 0.2, or 8.9 ± 0.2, or 9.0 ± 0.2, or 9.1 ± 0.2, or 9.2 ± 0.2, or 9.3 ± 0.2, or 9.4 ± 0.2, or 9.5 ± 0.2, or a lower, intermediate, or higher value.

Recognizing that pH values approaching and exceeding 9.0 ± 0.2 impose an increasing risk of destabilizing full AAV capsids, elution pH may be moderated by addition of salt. For example, in place of elution with a step or gradient to pH 9.5 ± 0.2, elution may be alternatively conducted with a step or gradient to pH 8.5 ± 0.2 in the presence of 2 mM NaCl, or 5 mM NaCl, or 10 mM NaCl, or 15 mM NaCl, or 25 mM NaCl, or a lower, intermediate, or higher value. Alternatively, the step or pH gradient can be conducted at pH 8.0 ± 0.2, or at pH 7.5 ± 0.2, or at pH 7.0 ± 0.2, or at pH 6.5 ± 0.2, or at pH 6.0 ± 0.2, or at pH 5.5 ± 0.2, or a lower, intermediate or higher value augmented by the addition of salt. It will be evident to persons of skill in the art that the more salt added, the lower the pH can be. If desired, the amount of salt added at any pH value can be increased to any value within the range of 2.5 mM to 250 mM, such as 2.5 mM, or 5 mM, or 12.5 mM, 25 mM, or 50 mM, or 100 mM, or 150 mM, or 200 mM, or 250 mM, or lower, intermediate, or higher value.

Any of the above embodiments may be performed in the presence of divalent metal cations such as magnesium and/or calcium, where the concentration of either is in the range of 0.1 mM to 10 mM, or 0.5 mM to 5 mM, or 1.0 mM to 2.75 mM, or 1.5 mM to 2.5 mM, or 1.75 mM to 2.25 mM, or 2.0 mM ± 0.1 mM, or a higher value such as up to 5 mM.

In one embodiment, the inclusion of divalent metal cations such as magnesium and/or calcium may be advantageous from an analytical perspective because the metal ions help to maintain the integrity of empty AAV capsids and tend to cause empty capsids to elute as a single peak. This makes it easier to measure the total amount of empty AAV capsids and the relative amounts of empty and full AAV capsids in a given sample.

In some embodiments, the presence of divalent metal cations such as magnesium and/or calcium may also be advantageous for preparative separations because they stabilize full capsids in addition to often improving their separation from empty capsids.

It is expected that the presence of magnesium will generally produce more desirable results with respect to AAV purity and/or recovery. Other embodiments may nevertheless include a deficiency or absence of divalent metal ions such as magnesium and/or calcium. This may particularly be the case where full AAV capsids exhibit adequate stability under the chosen separation conditions, and where the overall distribution of contaminants is such that fractionation in the absence of divalent metal cations enables isolation of a full capsid fraction with fewer contaminating empty AAV capsids compared to when the separation is conducted in the presence of such divalent cations.

Any of the foregoing embodiments may be performed in the presence of arginine or histidine for the purpose of stabilizing full capsids, and/or for improving capsid solubility, and/or suppressing non-specific interactions between capsids and the solid phase that might reduce separation efficiency or reduce full capsid recovery, and/or for simply reducing conductivity through the course of a separation

In some embodiments, arginine or histidine may be substituted directly in place of NaCI or other salts for performing gradient separations. Aqueous solubility of arginine is limited to about 600 mM and solubility of histidine is limited to about 200 mM. Arginine will be more convenient to work with because of its higher solubility but even the more limited solubility of histidine will allow it to be used effectively in many cases.

In embodiments where it is not possible to achieve an arginine or histidine concentration sufficient to elute the capsids, either may be combined with salts, for example including a combination of arginine plus sodium chloride or histidine plus sodium chloride. In some such embodiments, a baseline level of arginine can be added to the equilibration, wash, and/or elution buffers, or to only the wash and elution buffers, or only the elution buffer. Such a baseline level might be 25 mM or 50 mM, or at an intermediate concentration or higher concentration within its solubility range.

In another embodiment, arginine or histidine may be employed at baseline level across an ascending pH gradient, such as at a concentration 5 mM, or 10 mM, or at an intermediate concentration or higher concentration within its solubility range. Any of the above embodiments may be performed in the presence of low molecular weight zwitterions such as glycine or alanine or betaine. Such additives may have the effect of improving capsid solubility and stability without increasing conductivity. Such zwitterions elevate polarity but do not contribute to conductivity. They are also preferentially excluded from protein surfaces which allows them to confer a stabilizing effect. Such additives may be especially useful in conjunction with pH gradients to overcome the desolubilzing effect of low conductivity, where they may have the effect of improving peak sharpness and/or capsid stability.

In some embodiments, betaine may be employed from pH 5.0 to pH 10.0 while alanine and glycine will be restricted to a range from 5.0 to about 8.5. Above pH 8.5, the nitrogen atoms of glycine and alanine begin to lose their positive charge and the molecules becomes net electronegative. This causes them to contribute to the conductivity of the sample and also suspends other positive effects of their zwitterionic forms. The nitrogen atom on betaine is a quaternary amine that maintains its charge up to about pH 12, which keeps it in its zwitterionic form throughout that range.

In one embodiment where the operating pH is 8.5 or less, glycine or alanine may be included at a concentration up to 2 M or more, or 0.5 M to 2.5 M, or 1.0 M to 2.0 M, or 1.25 M to 1.75 M, or 1.4 M to 1.6 M, or about 1.5 M, or a different range.

In one embodiment, where the pH is less than 10, betaine may be included at a concentration up to 2 M or more, or 0.5 M to 2.5 M, or 1.0 M to 2.0 M, or 1.25 M to 1.75 M, or 1.4 M to 1.6 M, or about 1.5 M, or different range.

Any of the above embodiments may be performed in the presence of sugars such as intended to stabilize full capsids. Examples include but are not limited to sucrose, sorbitol, xylose, mannitol, trehalose, or other nonionic sugars at concentrations ranging from 0.5% to 25% or more.

Any of the above embodiments may be performed in the presence of glycerol such as intended to stabilize full capsids, for example at concentrations of 0.5% to 25% or more.

Any of the above embodiments may be performed in the presence of organic additives to minimize non-specific interactions between capsids and the surface of the primary amino solid phase. Such additives include surfactants, including non-ionic surfactants, at concentrations ranging from 0.01% to 1.0%. Such additives further include agents such as ethylene glycol or propylene glycol at concentrations ranging from 1% to 10%.

It will be recognized by persons of experience in the art that the method of the invention can be practiced on ligands other than primary amino groups that either entirely lack more heavily derivatized amines such as secondary, tertiary, or quaternary amines, or contain very low proportions of them. Likewise, it will be recognized that the results may be enhanced by the inclusion of hydrophobic residues or residues that mediate a larger increment of hydrogen bonding between capsids and the solid phase.

All references cited herein are incorporated by reference to the full extent to which the incorporation is not inconsistent with the express teachings herein.

The invention is further explained by the following non-limiting examples.

### Examples

### Example 1

Comparative separation of empty AAV capsids from full AAV capsids by salt elution of a quaternary amino solid phase versus a primary amino solid phase.

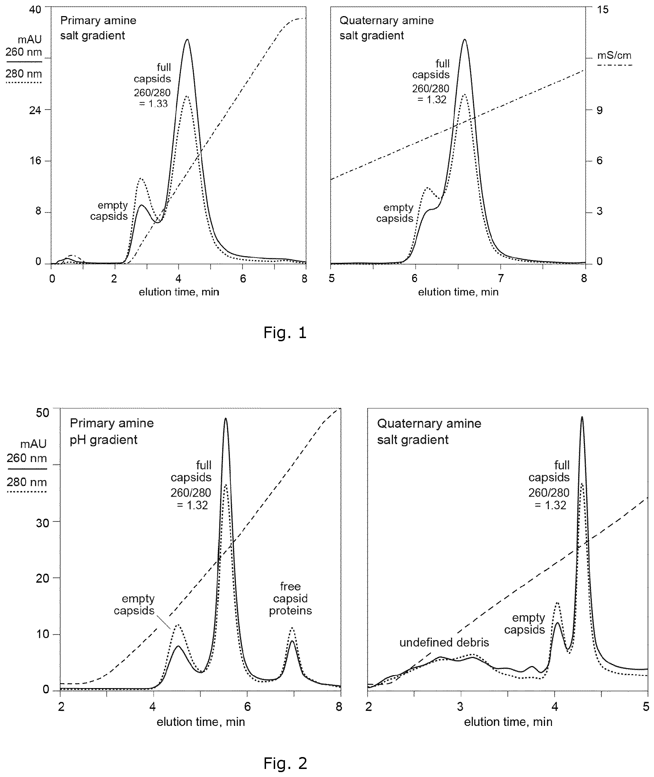

A 100-microliter monolith with a primary amine surface was equilibrated to 20 mM bis-tris-propane, 2 mM magnesium chloride, pH 8.8. A sample of cation exchange purified AAV 8 was applied to the column and washed with equilibration buffer. The monolith was then eluted with a 50 bed volume linear gradient ending at 20 mM bis-tris-propane, 2 mM magnesium chloride, 100 mM NaCl, pH 8.8. The same basic method was performed on a quaternary amine (strong anion exchange monolith, CIMac QA) except that the NaCI concentration in the gradient endpoint buffer was 200 mM. Neither column achieved complete separation of empty and full AAV capsids but the primary amine solid phase achieved better results (Figure 1).

### Example 2

Comparison of empty/full capsid separation with a primary amino solid phase eluted by ascending pH versus separation with a strong (quaternary amino) anion exchanger with a salt gradient.

A 100-microliter monolith with a primary amino surface was equilibrated to 10 mM bis-tris-propane, 10 mM Tris, 2 mM magnesium chloride, pH 8.0. A sample of cation exchange purified AAV 8 was applied to the column and washed with equilibration buffer. The column was then eluted with a 100 bed volume linear gradient ending at 10 mM bis-tris-propane, 10 mM Tris, 2 mM magnesium chloride, pH 9.5. The separation of empty and full AAV capsids was compared with a strong anion exchange (quaternary amine) monolith eluted with a salt gradient. The strong anion exchange monoliths was equilibrated with 20 mM bis-tris-propane, 2 mM magnesium chloride, pH 9.0. A sample of cation exchange purified AAV 8 was applied to the column and washed with equilibration buffer (same sample as used for the primary amine column experiment). The column was then eluted with a 50 column volume linear gradient ending at 20 mM bis-tris-propane, 2 mM magnesium chloride, 200 mM sodium chloride, pH 9.0. Results of the comparison are illustrated in Figure 2. As shown, elution of the primary amine column with an ascending pH gradient in the absence of excess salt was dramatically superior to the separation achieved by the strong anion exchanger with a salt gradient. In addition to demonstrating superior separation of empty and full capsids, the elution order of other contaminants demonstrate the fundamentally distinct selectivity of the primary amine column compared to the strong anion exchanger. Contaminants that eluted before the empty AAV capsids on the strong anion exchanger eluted after and better separated from the full AAV capsids on the primary amine column.

### Example 3

Removal of bound empty AAV capsids by a washing step in advance of pH gradient elution of full capsids.

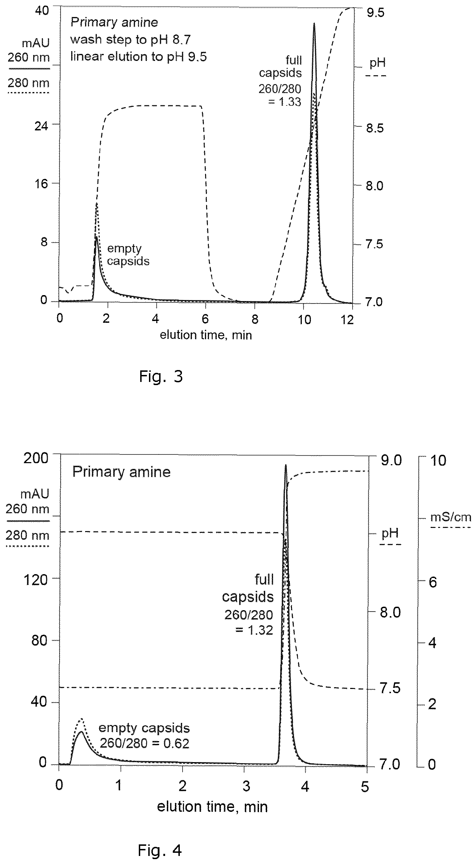

A primary amine monolith was equilibrated to 10 mM bis-tris-propane, 10 mM Tris, 2 mM magnesium chloride, pH 8.0. A sample of cation exchange purified AAV 8 was applied to the column and washed with equilibration buffer (same sample as used for Example 2). Empty AAV capsids were then removed with a washing step of 10 mM bis-tris-propane, 10 mM Tris, 2 mM magnesium chloride, pH 8.7. The column was then restored to its original equilibration conditions and eluted with a 40 bed volume linear gradient ending at 10 mM bis-tris-propane, 10 mM Tris, 2 mM magnesium chloride, pH 9.5. Results are illustrated in Figure 3. This experiment demonstrates the ability of the primary amine column to bind both empty and full AAV capsids at mildly alkaline pH (8.0) then support selective removal of empty AAV capsids with an ascending pH gradient (to pH 9.5), all in the absence of excess salts.

### Example 4

Equilibration under conditions where empty AAV capsids do not bind followed by elution of full AAV capsids with a single step that simultaneously increases salt concentration and lowers pH.

A primary amine monolith was equilibrated to 10 mM bis-tris-propane, 10 mM Tris, 2 mM magnesium chloride, pH 8.5. A sample of cation exchange purified AAV 8 was applied to the column and washed with equilibration buffer (same sample as used for Examples 2 and 3). Empty AAV capsids mostly failed to bind under these conditions and were almost entirely eliminated over the course of a long wash. Full AAV capsids were then eluted from the column with a step to 10 mM bis-tris-propane, 10 mM Tris, 2 mM magnesium chloride, 50 mM NaCl, pH 7.5. Results are illustrated in Figure 4. This experiment particularly documents the ability of the primary amine column to separate with empty AAV capsids from full AAV capsids with a highly simplified step gradient approach to accommodate even the simplest chromatography instrumentation.

### Example 5

The following examples illustrate the methodology of the method of the invention starting at pH 8 employing different ways to separate empty AAV capsids by increasing pH.

It is understood that capsids from different serotypes will respond differently to the same conditions, and that conditions will need to be adjusted individually for each different product. This series of Figures illustrates how easily capsids from any serotype may be screened to obtain the most effective conditions.

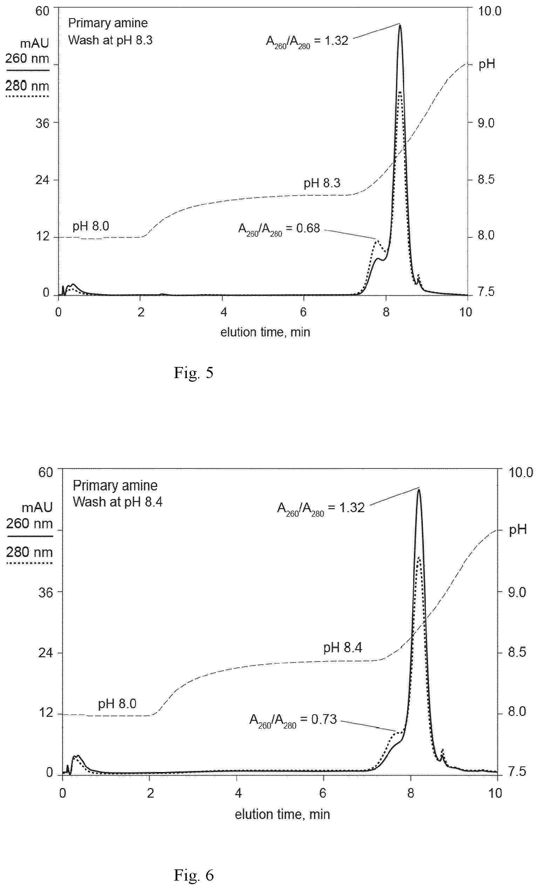

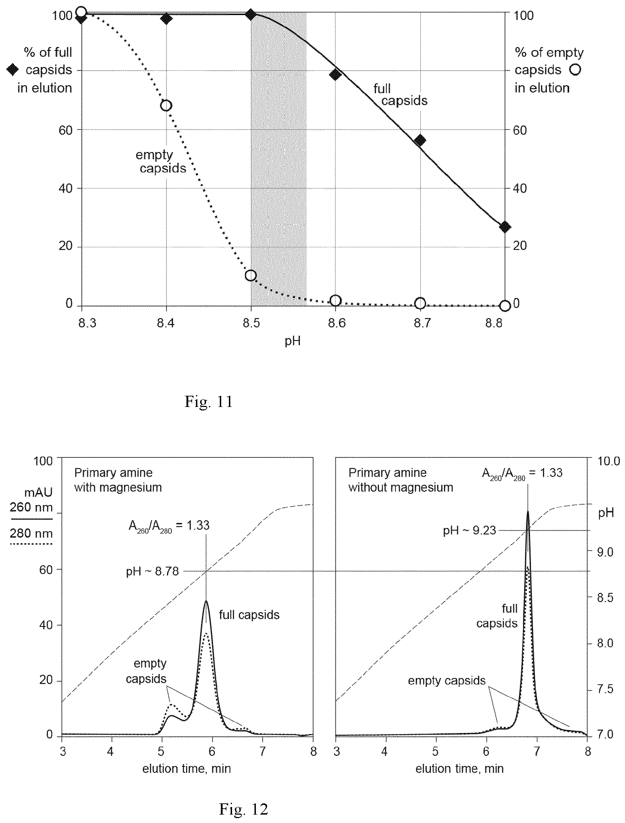

In a series of experiments sample containing empty and full AAV8 capsids was applied to a primary amino groups bearing solid phase at pH 8.0 (20 mM Tris, 20 mM Bis-Tris-Propane). In each experiment, the loaded column was washed with buffer at a higher pH (20 mM Tris, 20 mM Bis-Tris-Propane) and then eluted with an increasing pH gradient, ending at pH 9.5 (20 mM Tris 20 mM Bis-Tris-Propane). The original chromatograms are illustrated in the Figures (Fig. 5 - Fig. 10). Relative recovery of empty AAV capsids and full AAV capsids from the gradient is presented graphically in Fig. 11.

Fig. 5. Empty and full AAV capsids both bind at pH 8.0. Both remain bound throughout the wash at pH 8.3. Full capsids, as indicated by their UV absorbance ratio of 0.68, begin to elute immediately with application of the pH elution gradient. There is a definite but partial separation from the later eluting full capsids, which exhibit a distinct wavelength ratio of 1.32.

Fig. 6. Empty and full AAV capsids both bind at pH 8.0. Some of the empty AAV capsids are removed by the wash at pH 8.4. The remaining empty AAV capsids begin to elute immediately with application of the pH elution gradient. Full AAV capsids remain bound and elute in the pH gradient.

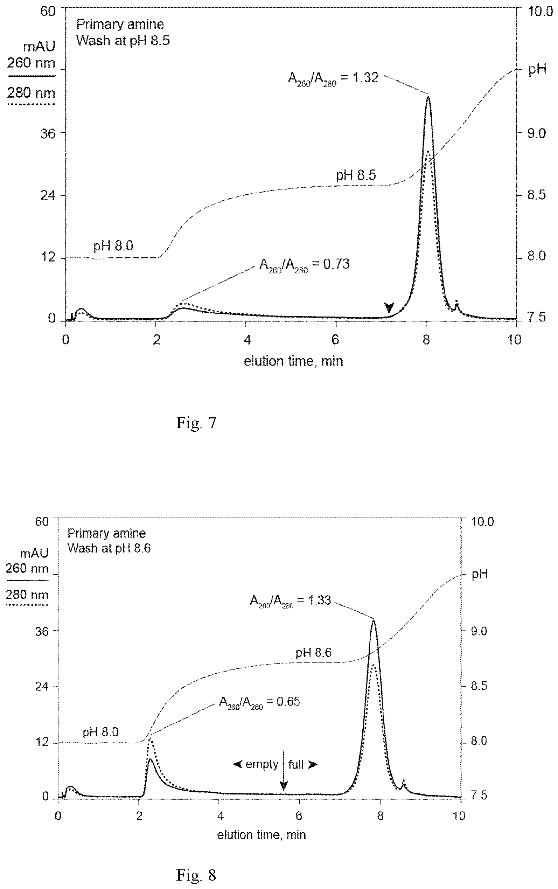

Fig. 7: Empty and full AAV capsids both bind at pH 8.0. Most of the empty AAV capsids are removed by the wash at pH 8.5. The remaining empty AAV capsids begin to elute immediately with application of the pH elution gradient. Full AAV capsids remain bound and elute in the pH gradient.

Fig. 8: Empty and full AAV capsids both bind at pH 8.0. All of the empty AAV capsids are removed by the wash at pH 8.6. Full AAV capsids remain bound and elute in the pH gradient.

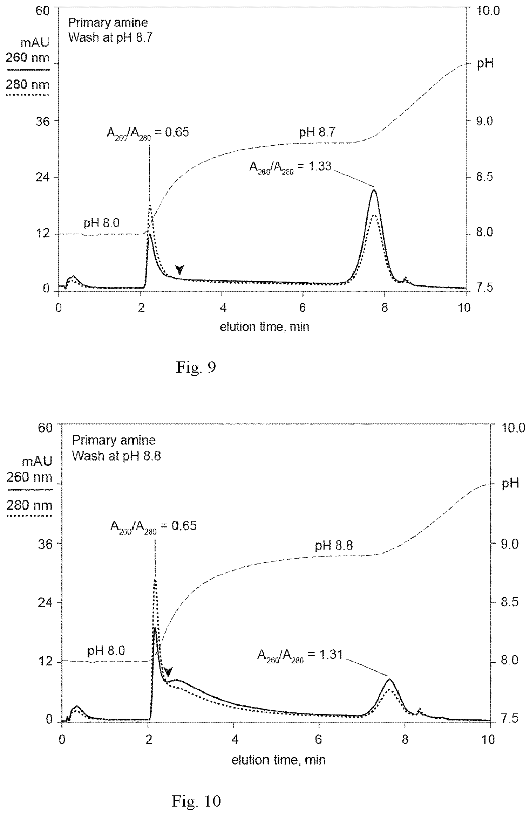

Fig. 9: Empty and full AAV capsids both bind at pH 8.0. All of the empty AAV capsids are removed by the wash at pH 8.7, however some full AAV capsids elute during the wash. The remaining full AAV capsids remain bound and elute in the pH gradient.

Fig. 10: Empty and full AAV capsids both bind at pH 8.0. All of the empty AAV capsids are removed by the wash at pH 8.6, however the majority of the full AAV capsids are also removed. The remaining full AAV capsids elute in the pH gradient.

Fig. 11: The graph illustrates the relative amounts of full and empty AAV capsids that elute in the gradient following wash steps at the various pH values. The shaded vertical bar indicates the wash pH values that support the most effective elimination of empty AAV capsids and best recovery of full capsids.

### Example 6

Alteration of selectivity by the inclusion or absence of magnesium ions.

Figure 12 compares the elution profiles between two chromatography experiments on a primary amine-bearing solid phase, where one profile was generated with 2 mM magnesium ions in both gradient buffers and the other profile was generated in the absence of magnesium ions. Otherwise, the buffers used for both experiments were the same. The equilibration buffer was 10 mM Tris, 10 mM bis-tris-propane, 15 mM NaCl, pH 7.0, (with or without 2 mM magnesium chloride). The gradient endpoint was 10 mM Tris, 10 mM bis-tris-propane, 15 mM NaCl, pH 9.5, (with or without 2 mM magnesium chloride). In the presence of magnesium, the full capsids eluted at about pH 8.77 (peak center). In the absence of magnesium, the full capsids eluted at about pH 9.23 (peak center), roughly a half pH unit higher than in the presence of magnesium. The separation of empty and full capsids was also more distinct in the presence of magnesium and the recovery of full capsids was about 50% higher.

### References

All references cited herein are incorporated by reference to the full extent to which the incorporation is not inconsistent with the express teachings herein.
[1] M. Lock, M. Alvira, J. Wilson, Analysis of particle content of recombinant adeno-associated virus serotype 8 vectors by ion exchange chromatography, Human Gene therapy Methods: Part B 23 (2012) 56-64.
[2] M. Lock, M. Alvira, Scalable purification method for AAV9, US patent application US20190002842A1, priority to US201562266357P, WO2017160360A9
[3] X. Fu, WC. Chen, C. Argento, P. Clarner, V. Bhatt, R. Dickerson, G. Bou-Assaf, M. Bahshaveshi, X. Lu, S. Bergelson, J. Pieracci, Analytical strategies for quantification of adeno-associated virus empty AAV capsids to support process development, Hum. Gene Ther. Met. 30 (2019) 144-152.

## Claims

1. A method for the separation or depletion of empty AAV capsids from full AAV capsids in an aqueous mixture comprising empty and full AAV capsids, wherein the mixture is contacted with a primary amino groups bearing solid phase surface in a first alkaline milieu whereby
(i) full AAV capsids bind to the solid phase surface whereas empty AAV capsids at least partially do not bind to the solid phase surface, and optionally bound empty AAV capsids are at least partially eluted by means of a second alkaline milieu of a pH value higher than the pH value of the first alkaline milieu, with the proviso that the second alkaline milieu does not elute full AAV capsids from the solid phase surface
or
(ii) both full and empty AAV capsids bind to the solid phase surface, and subsequently the empty AAV capsids are at least partially eluted by means of a second alkaline milieu of a pH value higher than the pH value of the first alkaline milieu, with the proviso that the second alkaline milieu does not elute full AAV capsids from the solid phase surface.

2. The method of claim 1 wherein the pH value of the first alkaline milieu is pH > 7 to pH 8.

3. The method of any one of the claims 1 or 2 wherein after the empty AAV capsids are at least partially eluted with the second milieu, the solid phase surface is contacted with a third alkaline milieu having a pH value greater than the pH value of the second milieu.

4. The method of claim 3 wherein the solid phase surface is contacted with a fourth alkaline milieu having a pH value less than the pH value of the third or second alkaline milieu but a higher salt concentration compared to the salt concentration of the first, second or third alkaline milieu.

5. The method of any one of the claims 1 to 4 wherein the pH value of the second alkaline milieu is in the range of pH 8.0 to pH 9.0, pH 8.1 to pH 8.9, pH 8.2 to pH 8.8, pH 8.3 to 8.7, or pH 8.4-8.6.

6. The method of any one of the claims 1 to 5 wherein the pH value of the third alkaline milieu is in the range of pH 8.5 to pH 10.5, or pH 8.5 to 10.0, or pH 8.5 to 9.5.

7. The method of any one of the claims 1 to 6 wherein the first, second, third and/or fourth alkaline milieu comprises a salt concentration corresponding to the concentration of up to 1 M of an alkali metal salt, such as NaCl, in particular 1 mM to 1,000 mM, or 2.5 mM to 250 mM

8. The method of any one of the claims 1 to 7 wherein the alkaline milieus comprise magnesium ions at a concentration in the range of 1.0 mM to 5.0 mM, in particular 1.5 mM to 3.0 mM, or 2.0 mM to 2.5 mM.

9. The method of any one of the claims 1 to 8 wherein the full and empty AAV capsids are of any serotype, in particular selected from the group consisting of natural and recombinant serotypes, chimeric, mixed, and combinations thereof.

10. The method of any one of the claims 1 to 9 wherein the primary amino groups bearing solid phase surface is arranged in a chromatography device.

11. The method of any one of the claims 1 to 10 wherein the primary amino groups bearing solid phase surface is a monolith, a column of packed particles, a column of packed nanofibers, a membrane adsorber, or a hydrogel.

12. Use of a solid phase extraction material having a solid phase surface bearing primary amino groups for the separation or depletion of empty AAV capsids in an aqueous mixture comprising empty and full AAV capsids.
